# EUROPÄISCHE PATENTANMELDUNG

(11) **EP 3 246 050 A1**
(43) Veröffentlichungstag der Anmeldung: **22.11.2017**
(21) Anmeldenummer: 16169905.3
(22) Anmeldetag: 17.05.2016
(51) Int. Cl.: A61L 15/26, A61L 15/42, A61L 15/44

(54) **WUND- ODER HAUTAUFLAGE**

(71) Anmelder: BSN medical GmbH, 20253 Hamburg (DE)
(72) Erfinder: ARSHI, Annahit, 22527 Hamburg (DE); HEMMRICH, Karsten, 40667 Meerbusch (DE); SCHULZE, Christian, 21255 Tostedt (DE)
(74) Vertreter: Jostarndt Patentanwalts-AG

(57) **Zusammenfassung**

Die vorliegende Erfindung betrifft eine pharmazeutische Zusammensetzung und hierbei insbesondere eine Haut- oder Wundauflage, die ein sauer-hydrolysierendes Oligomer und/oder Polymer, und ein säure-aktivierbares Prodrug umfasst, so dass in der pharmazeutischen Zusammensetzung bzw. in der Haut- oder Wundauflage der Wirkstoff durch Säureeinwirkung aus dem Prodrug gebildet wird. Die Erfindung betrifft ferner ein entsprechendes Verfahren zur Freisetzung von Wirkstoffen aus einer pharmazeutischen Zusammensetzung.

## Beschreibung

Die vorliegende Erfindung betrifft eine pharmazeutische Zusammensetzung und hierbei insbesondere eine Haut- oder Wundauflage, die ein sauer-hydrolysierendes Oligomer und/oder Polymer, und ein säure-aktivierbares Prodrug umfasst, so dass in der pharmazeutischen Zusammensetzung bzw. in der Haut- oder Wundauflage der Wirkstoff durch Säureeinwirkung aus dem Prodrug gebildet wird. Die Erfindung betrifft ferner ein entsprechendes Verfahren zur Freisetzung von Wirkstoffen aus einer pharmazeutischen Zusammensetzung.

Aus dem Stand der Technik sind zahlreiche Haut- und Wundauflagen zur Behandlung von Wunden oder Hauterkrankungen bekannt. Die Haut- oder Wundauflagen müssen hierbei verschiedene Anforderungen erfüllen, die aufgrund der Unterschiedlichkeit der zu behandelnden Wunden oder Hauterkrankungen hohe Anforderungen an die Haut- und Wundauflagen stellen. So muss bei stark extrudierenden Wunden wie Brandwunden die aus der Auflage austretende Flüssigkeit aufgenommen werden, wobei ein Flüssigkeitsfilm auf der Wunde verbleiben soll. Die Auflage muss weiterhin das Eindringen von Fremdkörpern oder Erregern in die Wunde verhindern. Sie darf nicht mit der Wunde verkleben oder zu Reizzuständen in dem bedeckten Gewebe führen. Neuere Entwicklungen sehen die Freisetzung von Medikamenten und/der wundheilungsaktiven Substanzen vor, die gerade bei chronischen Wunden, durch Behandlung der zugrundeliegenden Erkrankung eine erhebliche Therapieverbesserung darstellen. Bei derartigen Auflagen unter Wirkstofffreisetzung ist es von entscheidender Bedeutung, dass der Wirkstoff in kontrollierter Form freigesetzt wird.

Gemäß der EP 0 481 042 A1 kann ein Wirkstoff aus einer Hautauflage in kontrollierter Weise durch lontophorese freigesetzt werden, wobei ein aktives Elektrodenelement, eine wirkstoffhaltige Trägerschicht und eine Hydrogelschicht als Hautkontaktschicht verwendet wird. Eine derartige Applikationsform weist zahlreiche Nachteile auf. So ist sie als elektrisch gesteuerte Applikationsform komplex aufgebaut und teuer in der Herstellung. Da das Ausmaß der Wirkstoff-Freisetzung von dem ausreichenden Hautkontakt und der gleichbleibenden Leitfähigkeit abhängig ist, ist sie in der Anwendung zudem sehr fehleranfällig.

Als alternative Strategie offenbart die WO 2009/054006 A2 eine Wundauflage mit einem Gerüst aus einem abbaubaren Polymer, in das der Wirkstoff eingekapselt ist. Durch den hydrolytischen Abbau wird das Polymergerüst zerstört und der Wirkstoff freigesetzt. Diese Systeme sind komplex in ihrer Herstellung und nur bedingt für die dermatologische Anwendung geeignet, da der für die Freisetzung notwendige starke Abbau des Polymergerüsts meist erst nach längerer Zeitdauer vonstatten geht.

Es besteht daher noch Bedarf an neuen Haut- und Wundauflagen, die in kontrollierter Weise pharmazeutische Wirkstoffe freisetzen.

Aufgabe der Erfindung ist es daher eine Haut- oder Wundauflage bereitzustellen, die bezüglich mindestens einer der oben genannten Nachteile verbessert ist.

### Zusammenfassung der Erfindung

Diese Aufgabe wird gemäß der Erfindung dadurch gelöst, dass eine Haut- oder Wundauflage bereitgestellt wird, die folgendes umfassend:
(a) ein Oligomer und/oder Polymer, das durch seine Hydrolyse ein saures Milieu erzeugen kann, und
(b) ein säure-aktivierbares Prodrug,
wobei das säure-aktivierbare Prodrug durch die mittels Hydrolyse gebildete Säure in der Haut- oder Wundauflage aktivierbar ist.

Die erfindungsgemäße medizinische Auflage vereinigt mehrere entscheidende Vorteile gegenüber den aus dem Stand der Technik bekannten therapeutischen Ansätzen.

Es zeigte sich in überraschender Weise, dass die säurebildende Hydrolyse von Oligomeren oder Polymeren innerhalb einer solchen Haut- oder Wundauflage in zuverlässiger Weise die kontrollierte Generierung von Wirkstoffen aus einem säureaktivierbaren Prodrug ermöglicht und eine entsprechend kontrollierte Freisetzung dieser Wirkstoffe nach sich zieht.

Da es sich hier um ein modular aufgebautes Freisetzungssystem mit Oligomer und Polymer auf der einen Seite und säure-aktivierbarem Prodrug auf der anderen Seite handelt, kann man unter Auswahl der entsprechenden Komponenten das System gezielt an die jeweiligen Anforderungen anpassen.

Durch die Einkapselung des Prodrugs in die Polymermatrix kann die Säure-induzierte Wirkstoffgenerierung hierbei mit der durch Degradation induzierten Freisetzung des Prodrugs kombiniert werden und so ganz neuartige Freisetzungsprofile erzielt werden.

Da die Haut- oder Wundauflage initial nur das Prodrug, nicht aber den Wirkstoff selber enthält, ist eine vorzeitige unerwünschte Freisetzung des Wirkstoffs ausgeschlossen.

Im Gegensatz zu den Systemen mit einer degradierbaren Polymerträgermatrix muss das Oligomer oder Polymer hier nicht unbedingt als strukturbildendes Element wirken, es kann daher zahlreiche Ausgestaltungen annehmen, die von Partikeln über Filmen bis hin zu Fasern reicht.

Bei dieser lediglich auf die katalytische Funktion der Oligomere und Polymere abzielenden Ausgestaltung kann in vorteilhafter Weise auf die bestehenden Gestaltungen zu Haut- oder Wundauflagen zurückgegriffen werden, was eine schnelle und einfachere Entwicklung ermöglicht.

Alternativ kann das Säure-generierende Polymer in seiner Ausgestaltung als Faser oder Film durchaus selber als strukturbildendes Element wirken, so dass auf weitere Matrixbestandteile verzichtet werden kann.

Da die Hydrolysierbarkeit von Oligomeren und Polymeren ein breites Reaktionsspektrum umfasst, können unterschiedlichste Freisetzungsraten realisiert werden. Zudem gibt es auch Enzyme wie Lipasen oder Esterasen, durch deren Zugabe sich die Hydrolyserate weiter erhöhen lässt.

Aus dem Stand der Technik sind zahlreiche biokompatible und sauer hydrolysierende Polymere bekannt, wie beispielsweise Polymilchsäure (PLA) oder Polyhydroxyessigsäure (PGA), auf die der Fachmann hier zurückgreifen kann.

Die erfindungsgemäße Lösung bietet zudem den Vorteil, dass sie eine Plattform-Technologie darstellt, insofern sie auch für andere pharmazeutische Zusammensetzungen wie Cremes, Tabletten oder Dragees anwendbar ist.

Aufgrund der hochkontrollierten Steuerung der Freisetzung können auch Haut- oder Wundauflagen erzeugt werden, die den Wirkstoff nur in sehr geringen Mengen freisetzen. Dies ist insbesondere bei hochpotenten Wirkstoffen ein entscheidender Vorteil. Zudem erlaubt dies die Entwicklung der Haut- und Wundauflage als Medizinprodukt (z.B. als sog. Medical device class III), insofern hier eine Wundauflage vorliegt, bei der die Wirkung primär durch die mechanischen oder physikalischen Eigenschaften der Auflage bedingt ist.

Durch einfache Anpassung der Haut- oder Wundauflage bezüglich Größe, Form und Material kann sie gezielt an die Behandlungserfordernisse angepasst werden.

So kann sie in Verbindung mit einer pH-labilen Stickstoffmonoxid-Vorstufe als Prodrug zur Freisetzung von hochreinem NO dienen, welches ansonsten nur unter erheblichen Aufwand herzustellen ist. Auf diese Weise kann auch auf eine externe Zuführung von NO verzichtet werden.

Bei der erfindungsgemäßen Auflage handelt es sich um eine einfach aufgebaute medizinische Auflage mit handelsüblichen Komponenten, so dass es nicht nur kostengünstig sowie auf leichte Art und Weise herstellbar ist, sondern auch bei geringer Fehleranfälligkeit einfach in der Anwendung ist.

Die mit dem Herstellungsverfahren erzeugten Haut- und Wundauflagen eröffnen im Hinblick auf die kennzeichnenden Parameter und auf die Materialauswahl weitere Freiheitsräume.

Die erfindungsgemäße Wundauflage ist für ein breites Spektrum an Wunden verwendbar und insbesondere für die Behandlung chronischer Wunden geeignet. Die Bereitstellung eines aziden pH-Milieus erfolgt in der Auflage unabhängig von dem pH-Wert der Wunde. So weisen beispielsweise chronische Wunden im Allgemeinen einen pH-Wert von > 7 auf und sind damit nicht in der Lage eine aufliegende Wundauflage anzusäuern. Durch die intrinsische Azidifizierung kann die erfindungsgemäße Wundauflage aber auch bei einem alkalischen Wundmilieu ihren Zweck erfüllen.

Die Ansäuerung der Wundauflage kann auch zu einer Ansäuerung des Wundraums führen. Da ein pH-Wert von < 6 und insbesondere ein pH zwischen 4 und 5 mit einer erheblichen Reduzierung an humanpathogenen Bakterien einhergeht, kann die erfindungsgemäße Wundauflage hierdurch auch antibakteriell wirken und hier eine zusätzlichen positiven Effekt hervorrufen.

Zusammenfassend stellt die erfindungsgemäße Haut- oder Wundauflage eine Therapieform dar, bei der preiswert, zuverlässig, sicher und für den Patienten individualisierbar ein pharmazeutischer Wirkstoff von einer medizinischen Auflage freigesetzt wird.

### Die Erfindung im Einzelnen

Im Rahmen der Erfindung ist unter einer Haut- oder Wundauflage jegliche flächenförmige Vorrichtung zu verstehen, die auf Körperbereiche aufgelegt werden kann. Das Auflegen umfasst hierbei ein einfaches Auflegen ohne engen oder adhäsiven Kontakt, aber auch ein zumindest teilweise adhäsive Verbindung der Auflage mit der Haut. Eine solche adhäsive Verbindung oder Klebeverbindung ist zweckmäßigerweise als reversible Klebeverbindung ausgestaltet.

Im Rahmen der Erfindung ist unter einem Oligomer ein Molekül zu verstehen, das aus mehreren strukturell gleichen oder ähnlichen Einheiten aufgebaut ist. Bei einer größeren Anzahl von Einheiten (< 20 Einheiten) spricht man von einem Polymer.

Ein Oligomer oder Polymer, das durch Hydrolyse ein saures Milieu erzeugen kann, ist erfindungsgemäß dadurch gekennzeichnet, dass die Hydrolyseprodukte in ihrer Gesamtheit eine Ansäuerung des vorhandenen pH-Wertes bewirken. So entsteht bei der Hydrolyse eines Polyesters jeweils ein Molekül mit einer freien Carbonsäuregruppe und einer Hydroxylgruppe, so dass durch die freigesetzte Carbonsäure eine Ansäuerung des Milieus erfolgt. In bevorzugter Weise wird bei der Oligomer- oder Polymerhydrolyse das Polymerrückgrat hydrolysiert. Diese fallen dann unter die Klasse der sog. biodegradierbaren Polymere bzw. Oligomere. Es können aber auch Seitenketten des Oligomers oder Polymers, z.B. als Ester- oder Anhydridseitenketten zur einer sauren Hydrolyse führen.

Als Prodrug wird im Kontext der Erfindung ein inaktiver oder wenig aktiver pharmakologischer Stoff bezeichnet, der erst durch eine chemische Umwandlung in einen aktiven bzw. aktiveren Wirkstoff überführt wird. Die chemische Umwandlung kann eine Umlagerung, eine Anlagerung eines Atoms oder Moleküls an das Prodrug oder in bevorzugter Weise eine Spaltung des Prodrugs sein. Unter dem säure-aktivierbaren Prodrug ist erfindungsgemäß ein Prodrug zu verstehen, bei dem die chemische Umwandlung in den Wirkstoff durch Versetzen mit einer Säure (als einem Protonendonator gemäß der Definition von Brønstedt und Lowry) initiiert wird. Dies bedeutet insbesondere, dass diese chemische Umwandlung erst im sauren Milieu (pH < 7) initiiert wird.

In der einfachsten Ausführungsform wird das in der Haut- oder Wundauflage enthaltenden Oligomer oder Polymer durch die in der Anwendung in die Auflage eindringende wässrige Flüssigkeit (z.B. in Form des Wundexsudats) hydrolysiert.

Hierbei kommen zweckmäßigerweise Oligomere oder Polymere zur Anwendung, die eine hohe Hydrolyseempfindlichkeit aufweisen. Beispiele hierfür sind die zyklischen Ester-Dimere Glycolid und Lactid.

Bei einem sauren Wundexsudat wird die Hydrolyse im Rahmen einer Säure-katalysierten Hydrolysereaktion (z.B. bei Anhydriden und Estern) weiter verstärkt. So weisen z.B. putride Wunden (d.h. mit Fäulnisbakterien wie Clostridien befallene Wunden) einen stark sauren pH-Wert auf, der eine besonders effektive Hydrolyse des Oligomers oder Polymers und damit mit einer erhöhten Freisetzung des Wirkstoffs einhergeht.

Zudem kann die Hydrolyse innerhalb der Auflage auch durch vom Körper aufgenommene Enzyme initiiert oder verstärkt werden. So ist bekannt, dass die in entzündeten Wunden vorhandenen Makrophagen beispielsweise das Enzym Cholesterol-Esterase freisetzen, welches zusammen mit dem Wundexsudat in die Wundauflage eindringen kann und dort zu einer Esterhydrolyse führen kann.

Durch die erfindungsgemäße Haut- oder Wundauflage findet eine Umwandlung des Prodrugs in den aktiven Wirkstoff bereits innerhalb der Haut- oder Wundauflage statt, so dass von der Haut- oder Wundauflage der aktive Wirkstoff freigesetzt wird. Damit wird die bei Prodrugs übliche Metabolisierung im Organismus durch die Auflage selber vorweggenommen.

Hierdurch können auch Prodrugs erstmalig zur Anwendung kommen, die als solche nicht für eine Aufnahme in den Organismus geeignet sind, weil sie bspw. eine mangelhafte Pharmakokinetik besitzen oder toxisch sind.

In einer bevorzugten Ausführungsform umfasst die Haut- oder Wundauflage zusätzlich einen Induktor und/oder Katalysator der Oligomer- oder Polymer-Hydrolyse.

Als Induktor ist im Sinne der Erfindung hierbei Wasser oder eine wässrige Flüssigkeit anzusehen, die als essentieller Reaktionspartner der Hydrolyse benötigt wird.

Die erfindungsgemäße Haut- oder Wundauflage muss hierbei so ausgestaltet sein, dass das Wasser oder die wässrige Feuchtigkeit erst zum Zeitpunkt der Anwendung in Kontakt mit den Oligomeren oder Polymeren kommt. Dies kann beispielsweise durch eine wasserundurchlässige Umverpackung gewährleistet werden, so dass erst bei der Anwendung durch die Luftfeuchtigkeit bzw. Hautfeuchtigkeit und/oder das Wundexsudat die Hydrolyse initiiert wird.

Alternativ kann das Wasser bzw. die wässrige Flüssigkeit innerhalb der Wundauflage in einem von dem Oligomer- oder Polymer getrennten Kompartiment vorliegen, so in einer gesonderten Schicht der Auflage oder in verkapselter Form.

Als Katalysator hierbei jeglicher Stoff anzusehen, die die Hydrolysegeschwindigkeit erhöht, ohne dabei selbst verbraucht zu werden. Er kann somit die Hydrolyse initiieren oder eine bereits initiierte Hydrolyse beschleunigen. Als Katalysatoren können anorganische, organische Substanzen oder Biomoleküle dienen. Der einfachste Katalysator stellt hierbei das Proton bzw. Hydroxoniumion dar, der bei der Hydrolyse von Estern oder Anhydriden durch Protonierung des Carbonyl-O-Atoms die Carbonylgruppe aktiviert und eine nucleophile Addition von Wasser erleichtert.

Im Bereich der Biomoleküle können im Rahmen der Erfindung insbesondere Enzyme als Katalysatoren verwendet werden.

Dem Fachmann stehen für die einzelnen zu hydrolysierenden Oligomere oder Polymere zahlreiche Enzyme zur Verfügung, die er entsprechend den jeweiligen Anforderungen einsetzen wird. Bei Oligoestern oder Polyestern wird zweckmäßigerweise eine Esterase oder Lipase als Enzym eingesetzt.

Bevorzugte Enzyme sind dabei unter den folgenden ausgewählt: Schweineleberesterase oder Isoformen hiervon, Acetyleterase, Butyrylcholinesterase Acetylcholinesterase, *Rhyzopus oryzae* Esterase, *Bacillus stearothermophilus-Esterase, Saccharomyces cerevisiae*-Esterase, *Bacillus subtilis*-Esterase, Pektin-Esterase, Proteinase K, Schweinepankreas-Lipase, *Candida rugosa*-Lipase, *Candida antartica*-Lipase, *Pseudomonas fluorescens*-Lipase.

Insbesondere bevorzugt ist hierbei die Schweineleberesterase (PLE) oder ein PLE-Isoenzym wie PLE-2, PLE-3, PLE-4, PLE-5 oder PLE-6.

In einer Ausführungsform der Erfindung ist das unter Säurebildung hydrolysierende Polymer ausgewählt aus der Gruppe enthaltend Polyester, Polyanhydrid und Polyorthoester, wobei ein Polyester bevorzugt ist.

Als Polyester sind hierbei die aliphatischen Polyester bevorzugt, die sich in zwei Klassen einteilen lassen, die erfindungsgemäß beide angewendet werden können.

Die erste Klasse besteht aus den Polyhydroxyalkanoaten. Diese Polymere sind aus Hydroxyalkansäuren (auch als Hydroxyfettsäuren bezeichnet) HO-R-COOH aufgebaut. Beispiele hierfür sind Polyhydroxymilchsäure (Polylactid, PLA) oder Polyhydroxyessigsäure (Polyglycolid, PGA).

Die zweite Klasse der sogenannten Poly(alkyldicarboxylate) wird durch Polykondensation aus Diolen und Dicarbonsäuren gebildet. Beispiele hierfür sind Poly(butylensuccinat) oder Poly(ethylensuccinat).

Bevorzugte Polyester sind dabei unter den folgenden ausgewählt: Poly(D,L-lactid), Poly(D-lactid), Poly(L-lactid), Poly(D,L-glycolid) (PDLLA), Poly(D-glycolid), Poly(L-glycolid), Poly(lactid-Co-Glycolid) (PLGA), Polyhydroxybutyrat, Poly(ε-caprolacton) (PCL), Poly(ε-caprolacton-Co-Glycolid-Co-DL-Lactid), Poly(p-Dioxanon) (PPDO), Poly(butylensuccinat), Poly(ethylensuccinat) (PES), Poly(butylensuccinat-Co-adipat), Ply(trimethylencarbonat), Poly(propylenfumarat) (PPF) und andere aliphatischen Polyester sowie deren Copolymere einschließlich segmentierter Blockcopolymere aus Polyether und Polyestersegmenten, wie sie bspw. aus der Umsetzung von hochmolekularen Polyestern mit hydroxyterminierten Poly(alkylenglykolen) erhalten werden können.

Als Polyester sind hierbei bevorzugt PGA, PLA oder PGLA.

Des Weiteren können als Polymere Polyanhydride eingesetzt werden. Polyanhydride sind Polymere mit der folgenden Grundstruktur:

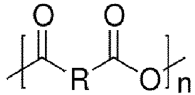

Erfindungsgemäß können sowohl aliphatische, sowie ungesättigte und aromatische Polyanhydride verwendet werden. Bevorzugte Polyanhydride sind hierbei ausgewählt aus der Gruppe umfassend Poly(sebacinsäure), Poly(adipinsäure), Poly[1,3-bis(p-Carboxyphenoxy) propan:Sebacinsäure), Poly(Fettsäuredimer:Sebacinsäure) Copolymer p(FAD:SA), Poly(glycerolsebacinsäure) (PGS), und den in den folgenden Patentschriften offenbarten Verbindungen U.S. Nr. 4,757,128; 4,997,904; 4,888,176; 4,857,311; und Nr. 5,264,540.

Geeignete Polyanhydride sind kommerziell erhältlich wie bspw. Gliadel® - ein Copolymer aus 20 mol-% 1,3-bis(p-Carboxyphenoxy)-propan)- Einheiten und 80 mol-% Sebacinsäure- Einheiten.

In einer weiteren Ausführungsform werden als Polymere Polyorthoester eingesetzt. Es handelt sich hierbei um Polymere mit der folgenden Grundstruktur:

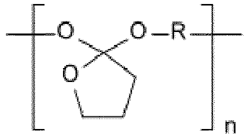

Die Polyorthoester umfassen die wie folgt dargestellten vier Strukturklassen POE I-IV, die alle im Rahmen der Erfindung verwendet werden können:

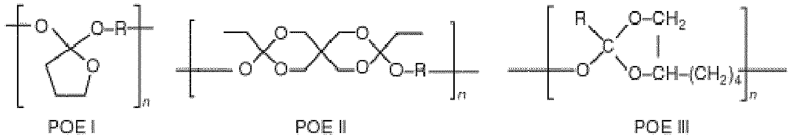

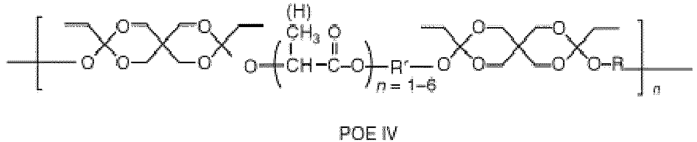

In einer weiteren Ausführungsform kann das Polymer ein Polymer mit hydrolysierbaren Seitenketten sein. Diese sind bevorzugt Ester- oder Anhydridgruppen. Beispiele für derartige Polymere sind: Acetyliertes Pectin, Acetyliertes Polyethylenimin oder acetyliertes Chitosan. Diese Polymere besitzen den Vorteil, dass unabhängig vom Polymerrückgrat die Hydrolysierbarkeit über den Anteil der Estergruppen (der Anhydridgruppen) gesteuert werden kann. Zudem werden zum Abbau keine Endo-Hydrolasen als Enzyme benötigt, sondern bei acetylierten Polymeren können die Acetyl-Esterasen eingesetzt werden.

Als Oligomere können zweckmäßigerweise die entsprechenden kurzkettigen Analoga der oben aufgeführten Polymere eingesetzt werden. Ein Beispiel hierfür ist das Essigsäureanhydrid.

In einer bevorzugten Ausführungsform wird ein zyklisches Oligomer und besonders bevorzugt ein zyklischer Ester oder ein zyklisches Anhydrid eingesetzt. Bevorzugte Beispiele sind hierbei Glycolid und Lactid.

Diese zyklischen Oligomere weisen häufig eine besonders hohe Hydrolyseempfindlichkeit auf, so dass sie auch ohne katalytische Unterstützung in ausreichender Weise ein saures Milieu erzeugen können.

Die Haut- oder Wundauflage kann auch so ausgestaltet sein, dass neben dem Wirkstoff auch die Hydrolyseprodukte freigesetzt werden.

So kann durch Freisetzung der Säure ein für Wundheilung vorteilhaftes saures Milieu im Bereich der Wunde erzeugt werden, da hierbei durch die Reduktion der absoluten Bakterienmenge ein wesentliches Therapieziel in der modernen Wundbehandlung erreicht werden kann. So werden die meisten humanpathogenen Keime in ihrem Wachstum in einem pH-Wert < 6,0 und insbesondere zwischen pH 4-5 gehemmt, wohin deren pH-Optimum mit 6,2 - 7,8 angegeben wird. Entsprechend konnte bei Patienten mit Hemiplegie oder Diabetes mellitus gezeigt werden, dass nach Senkung des pH-Wertes der intakten Haut durch topische Verwendung von sauren Externa die Bakteriendichte deutlich reduziert wird.

Diese bakterizide Wirkung der freigesetzten Säure kann in vorteilhafter Weise durch die Freisetzung des Alkohols als dem zweitem Reaktionsprodukt einer Esterhydrolyse unterstützt werden.

Das sauer abbauende Polymer kann jede für Polymer bekannte Form annehmen, so kann es als Gel, Partikel, Folie, Film, Schaum, oder als textiles (Flächen)gebilde ausgebildet sein.

In einer bevorzugten Ausführungsform der Erfindung ist bei der Haut- oder Wundauflage das sauer hydrolysierende Polymer als Faser oder als Hüllschicht einer Kernfaser ausgeformt.

Die Kernfaser besteht hier bevorzugt aus einem Thermoplasten, der in bevorzugterweise in seinem Spinnverhalten ähnlich oder identisch mit dem Hüllschicht bildenden sauerabbauenden Polymer ist, die Materialien somit spinnkompatibel sind.

Durch Auswahl eines geeigneten Faserquerschnitts hat der Fachmann eine zusätzliche Möglichkeit, die Abbaubarkeit des Polymers zu steuern. Beispielhaft seien hier die folgenden Faserquerschnitte genannt: flach, oval, rund, dreieckig, dreilappig, Y-förmig, T-förmig, M-förmig, S-förmig, Y-förmig, H-förmig, sternförmig oder schneeflockenartig.

Für die Verarbeitung der erfindungsgemäßen Polymerfasern stehen dem Fachmann im Prinzip sämtliche aus der Fasertechnologie bekannten Verarbeitungsarten zur Verfügung, so dass die Faser den Bestandteil eines Gewebes, Gewirkes oder Vliesstoffes bilden kann.

Diese entsprechenden Fasergebilde können gänzlich aus sauer abbauenden Polymerfasern bestehen, es können aber auch Mischgebilde eingesetzt werden, die zusätzlich oder überwiegend herkömmliche, d.h. nicht sauer-hydrolysierende Fasern enthalten.

In einer Ausführungsform kann das säureaktivierbare Prodrug selber einen Ester darstellen, der durch die Säure in die freie Carbonsäure als aktiven Wirkstoff umgesetzt wird.

In einer weiteren Ausführungsform ist das säureaktivierbare Prodrug ein deprotoniertes Säureanion, das durch die Säure in die unpolare Carbonsäure umgewandelt wird, die dann leichter in die Haut penetrieren kann.

In einer bevorzugten Ausführungsform ist das säureaktivierbare Prodrug eine pH-labile Stickstoffmonoxidvorstufe (NO-Vorstufe, NOD), die durch die Reaktion mit der Säure das Stickstoffmonoxid als pharmazeutischen Wirkstoff bildet.

pH-labile NO-Vorstufen sind im Stand der Technik bekannt und dem Fachmann geläufig.

In einer bevorzugten Ausführungsform der Erfindung sind die pH-labilen Stickstoffmonoxidvorstufen ausgewählt aus der Gruppe enthaltend organische Nitrate, anorganische Nitrate, Nitrite, Schwefel-, Stickstoff- oder Sauerstoff-Nitrosoverbindungen, NO-Metall-Verbindungen und NO-chelatierende Substanzen.

Beispiele für pH-labile NOD umfassen Diazeniumdiolate (z.B. US Patente Nr. 7,105,502; 7,122,529; 6,673,338), trans[RuCl([15]aneN4)NO]+2, Nitrosyl-Liganden, 6-Nitrobenzo[a]pyrol, S-Nitroso-Glutathion, S-Nitroso-Thiol, Nitroanilin-Derivate (siehe US 2013/0224083), 2-Methyl-2-Nitrosopropan, Imidazoyl-Derivate, Hydroxylnitrosamin, Hydroxylamin und Hydroxyharnstoff.

In bevorzugter Weise handelt es sich bei dem NOD um eine pharmakologisch verträgliche Substanz.

In einer bevorzugten Ausführungsform ist das NOD ein Nitritsalz. Bevorzugt sind hierbei Nitrite von Alkali- oder Erdalkalimetallen. Beispielhaft sind hier genannt: LiNO₂, NaNO₂, KNO₂, RbNO₂, CsNO₂, FrNO₂, Be(NO₂)₃, Mg(NO₂)₃, Ca(NO₂)₃, Sr(NO₂)₃, Ba(NO₂)₃, oder Ra(NO₂)₃. Die Konzentration der Nitritsalze bezogen auf das Gesamtgewicht der sie enthaltenden Schicht(en) kann hierbei bis zu 20 Gew.-% betragen, bevorzugt zwischen 0,25 und 10 Gew.-%, besonders bevorzugt zwischen 3 und 7,5 Gew.-%.

Besonders bevorzugt ist hierbei als NOD das NaNO₂, das in weiterhin bevorzugter Weise zusammen mit Ascorbat oder Askorbinsäure als Radikalfänger-System in der medizinischen Auflage enthalten ist.

Die Konzentration der Nitritsalze bezogen auf das Gesamtgewicht der sie enthaltenden Schicht(en) kann hierbei bis zu 20 Gew.-% betragen, bevorzugt zwischen 0,25 und 10 Gew.-%, besonders bevorzugt zwischen 3 und 7,5 Gew.-%.

In einer alternativen Ausgestaltung kann auch ein Nitratsalz verwendet werden, bei denen eine enzymatische Umwandlung in das entsprechende Nitritsalz möglich ist. Bevorzugt kommen hierbei Nitrate von Alkali- oder Erdalkalimetallen zur Anwendung. Beispielhaft sind hier genannt: LiNO₃, NaNO₃, KNO₃, RbNO₃, CsNO₃, FrNO₃, Be(NO₃)₃, Mg(NO₃)₃, Ca(NO₃)₃, Sr(NO₃)₃, Ba(NO₃)₃, oder Ra(NO₃)₃. Die Konzentration der Nitratsalze bezogen auf das Gesamtgewicht der sie enthaltenden Schicht(en) kann hierbei bis zu 20 Gew.-% betragen, bevorzugt zwischen 0,25 und 10 Gew.-%, besonders bevorzugt zwischen 3 und 7,5 Gew.-%.

In einer weiteren Ausführungsform der Erfindung können die NODs an ein Polymer oder Oligomer gekoppelt sein. Entsprechende Methoden zur Kopplung von NOD an Polymere sind beispielsweise durch das US-Patent Nr. 5,405,919 offenbart. In einer Ausführungsform handelt es sich bei dem NOD-gekoppelten Polymer um ein Polymer, das mit Diazoniumdiolatgruppen versehen ist.

In einer speziellen Ausführungsform der Erfindung sind die NODs an das erfindungsgemäße, sauer hydrolysierende Polymer oder Oligomer gekoppelt. Hierdurch werden die beiden grundlegenden Komponenten der erfindungsgemäßen Haut- oder Wundauflage in einer Substanz vereinigt.

Die Menge an freigesetztem NO beträgt zwischen 50 und 600 ppm und bevorzugt zwischen 160 und 400 ppm. Solche Mengen sind therapeutisch wirksam ohne zu gravierenden Nebenwirkungen zu führen.

In einer weiteren Ausführungsform der Erfindung liegt das säureaktivierbare Prodrug und hierbei insbesondere die NOD als Mikro- oder Nanopartikel vor.

Diese können in einer besonders bevorzugten Ausführungsform in der Polymermatrix eingebettet sein.

Um die bei der erfindungsgemäß bevorzugten NO-Generierung auftretenden mehrfach oxidierten Stickoxide, Sauerstoffradikalanionen oder Hydroxylradikale zu entfernen, ist es vorteilhaft, wenn die Haut- oder Wundauflage ein System umfasst, welches mehrfach oxidierte Stickoxide, Sauerstoffradikalanionen oder Hydroxylradikale abbaut oder neutralisiert, wobei das System bevorzugt ausgewählt ist aus der Gruppe enthaltend Ascorbinsäure, Vitamin E und Derivate hiervon, Thiole und Radikalfänger.

Die eingesetzten Substanzen des Radikalfänger-Systems können nicht nur die bei der NO-Generierung entstehenden radikalischen Nebenprodukte abfangen, sondern zudem dafür sorgen, dass die entsprechende Schicht sauerstoffarm oder sogar sauerstofffrei ist und damit auch eine initiale Reaktion des entstandenen NO mit dem Sauerstoff unterbunden wird.

In zweckmäßiger Weise liegen die pH-labilen NOD und das Radikalfänger-System in derselben Schicht vor. Dadurch können die bei der Acidolyse als Nebenprodukte entstehenden Radikale direkt abgefangen werden, ohne dass sie mit weiteren Substanzen unter Bildung von eventuell toxischen Substanzen abreagieren. Bevorzugterweise sind die NOD und das Radikalfänger-System in der mittleren Schicht enthalten.

In einer alternativen Ausführungsform, beispielsweise bei einer chemischen Inkompatibilität von NOD und Radikalfänger-System, liegen diese beiden Komponenten in unterschiedlichen Schichten vor. Hierbei ist es zweckmäßig, dass das NOD in der mittleren Schicht und das Radikalfänger-System in der inneren, d.h. der Haut zugewandten Schicht enthalten ist, so dass durch Acidolyse generierte NO mit seinen Nebenprodukten vor dem Auftreten auf die Haut bei dem Durchtritt durch die innere Schicht aufgereinigt wird.

In einer weiteren Ausführungsform umfasst die Haut- oder Wundauflage beschichtete Fasern, bei denen die Hüllschicht der Kernfaser ein Radikalfänger-System enthält. Durch die in der Hülle vorgesehenen Radikalfänger-Moleküle können toxische Nebenprodukte der NO-Bildung vor dem Austreten aus der Faser inaktiviert werden. Die NOD kann hierbei entweder in der Kernfaser oder in der Hüllschicht enthalten sein. In besonders bevorzugter Weise liegt das NOD in der Kernfaser und das Radikalfänger-System in der Hüllschicht vor. So können inkompatible Systeme miteinander kombiniert werden.

Bei dem "Radikalfänger-System" der vorgenannten Ausführungsformen handelt es sich bevorzugt um ein Antioxidans, und besonders bevorzugt um Ascorbat oder Ascorbinsäure. Ebenso bevorzugt ist hierzu eine Cu²⁺- Verbindung, insbesondere ein anorganisches Cu²⁺-Salz wie CuCl₂.

Dem Fachmann sind zahlreiche Systeme bekannt, welche in der Lage sind, mehrfach oxidierte Stickoxide, Sauerstoffradikalanionen, Hydroxylradikale oder aquatisierte Elektronen abzubauen oder zu neutralisieren. Diese wird er entsprechend der jeweiligen Schichtzusammensetzung der Haut- oder Wundauflage auswählen.

Für eine lipophile Haut- oder Wundauflage-Schicht, wie es durch ein hydrophobe Polymere bereitgestellt werden kann, eignen sich beispielsweise Antioxidantien wie Tocopherole, Tocotrienole, Tocomonoenole, Irganox®, Irgafos®, Butylhydroxyanisol (BHA) und Butylhydroxytoluol (BHT).

Für ein hydrophile Haut- oder Wundauflage-Schicht, eignen sich insbesondere organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und Phytoöstrogene.

In bevorzugter Weise enthält die Haut- oder Wundauflage und hierbei bevorzugt die Prodrug-haltige Schicht darüber hinaus einen oder mehrere der folgenden Stoffe: Katalysatoren, Detergentien, Puffersubstanzen, Chromophore, Substanzen, die das Prodrug stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

In einer bevorzugten Ausführungsform sorgt die Puffersubstanz dafür, dass bei einer zu starken Säurebildung der pH-Wert in einem für Wirkstoffgenerierung bzw. für das Hautmilieu optimalen pH-Bereich bleibt. So ist zu beachten, dass die bei der Esterhydrolyse gebildeten Säure die weitere Hydrolyse des Esters stimulieren kann und so einen positiven Feedback-Loop beinhalten kann. Die Puffersubstanz puffert hierbei zweckmäßigerweise in einem pH-Bereich zwischen 4 und 7, bevorzugt zwischen 4,5 und 6, und besonders bevorzugt zwischen 5 und 5,5.

Bei der Verwendung einer pH-labilen NO-Vorstufe als Prodrug kann bspw. Dimethylsulfoxid oder Ethanol als Stabilisator der NO-Vorstufe verwendet werden. Substanzen, die die Halbwertszeit von NO erhöhen werden, bspw. in der US 2003/0039697 offenbart.

In einer weiteren bevorzugten Ausführungsform beinhaltet das mehrschichtige Haut- oder Wundauflage und hierbei bevorzugt die Prodrug-haltige Schicht weiterhin einen Kristallisationsinhibitor. Verschiedene Tenside oder amphiphile Substanzen können als Kristallisationsinhibitoren verwendet werden. Sie sollten pharmazeutisch akzeptabel und für die Verwendung in Medikamenten geprüft sein. Ein besonders bevorzugtes Beispiel für einen solchen Kristallisationsinhibitor ist lösliches Polyvinylpyrrolidon, welches kommerziell erhältlich ist z.B. unter dem Handelsname Kollidon^{®} (Bayer AG). Andere geeignete Kristallisationsinhibitoren beinhalten Copolymere von Polyvinylpyrrolidon und Vinylacetat, Polyethylenglycol, Polypropylenglycol, Glycerol und Fettsäureester von Glycerol oder Copolymere von Ethylen und Vinylacetat.

Optional enthält die Haut- oder Wundauflage einen Penetrationsförderer. Solche Penetrationsförderer (auch "Permeationsenhancer") verbessern die Permeationseigenschaften für das Eindringen der pharmakologisch aktiven Substanzen in die Haut. Beispiele für Penetrationsförderer sind u.a. Fettalkohole, Fettsäuren, Fettsäureester, Fettsäureamide, Glycerin oder Glycerin-Fettsäureester, N-Methylpyrrolidon, Terpene wie Limonen, α-Pinen, α-Terpineol, Carvone, Carveol, Limonenoxid, Pinenoxid oder 1,8-Eukalyptol.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung als Haut- oder Wundauflage physiologische verträgliche und/oder dermatologisch verträgliche Stoffe und Stoffgemische zur Anwendung kommen.

In einer Ausführungsform der Erfindung enthält die medizinische Auflage und hierbei insbesondere die Prodrug-haltige Schicht eine oder mehrere pharmakologisch aktive Substanzen. Diese können die pharmakologische Wirkung des NOs unterstützen oder unabhängig von dem NO in einer für die entsprechende Anwendung therapeutisch relevanten Weise wirken.

In einer Ausführungsform der Erfindung enthält die medizinische Auflage eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

In einer bevorzugten Ausführungsform liegt die pharmakologisch aktive Substanz in Form von wachsartigen Partikeln mit einem niedrigen Schmelzpunkt vor, die bei Kontakt mit der Haut schmelzen und die Substanz freisetzen.

In einer weiteren Ausführungsform ist die Haut- oder Wundauflage und bevorzugt hierbei die NOD-haltigen Schicht sauerstoffarm oder sauerstofffrei. Entsprechenderweise beträgt der Sauerstoffgehalt der Haut- oder Wundauflage oder der NOD-haltigen Schicht weniger 2%, bevorzugt weniger als 1,5% und besonders bevorzugt weniger als 1% bezogen auf die Konzentration des NOD.

Diese erfindungsgemäße Sauerstoffarmut oder Sauerstofffreiheit kann durch Behandeln der Einzelkomponenten der Haut- oder Wundauflage oder Begasen von Zwischenstadien oder der fertigen Haut- oder Wundauflage mit einem inerten Gas (wie Argon oder Stickstoff) hervorgerufen werden. Ein solche Haut- oder Wundauflage ist zweckmäßigerweise gasdicht zu verpacken, so dass die Sauerstoffarmut bzw. Suaerstofffreiheit bis zum Zeitpunkt der Anwendung erhalten bleibt.

In einer weiteren Ausführungsform weist die Haut- oder Wundauflage und bevorzugt hierbei die NOD-haltigen Schicht(en) zur Erzielung der Sauerstoffarmut oder Sauerstofffreiheit einen Sauerstoffabsorber auf. Zu den geeigneten Sauerstoffabsorbern zählen: Irganox®, Irgafos®, Butylhydroxyanisol, Butylhydroxytoluol, Ascorbinsäure oder Pyrogallol.

In einer besonderen Ausführungsform umfasst die Wundauflage einen Träger, auf dem eine Schicht aus einem Gewirk oder Vlies angeordnet ist. Das Vlies oder Gewirk umfasst hierbei Fasern, die das sauer hydrolysierende Polymer oder Oligomer enthalten. Auf der der Wunde zugewandten Seite ist die Wundauflage bevorzugt mit einer Schutzschicht versehen, die für NO durchlässig ist und verhindert, dass das Gewirk oder das Vlies mit der Wunde verklebt.

Die mehrschichtige Haut- oder Wundauflage der vorliegenden Erfindung ist in einer Ausführungsform dadurch erhältlich, dass
(i) eine Lösung enthaltend NOD und mindestens ein hygroskopisches Polymer oder Copolymer flächig ausgestrichen und getrocknet wird und
(ii) die so erhaltene Schicht mit einer weiteren Schicht sowie mit einer Schicht, welche während der Behandlung für NO durchlässig wird, kaschiert wird.

In einer alternativen Ausführungsform wird im Schritt (i) die NOD-haltige Lösung auf eine Vlies, ein Gewirk oder ein hygroskopisches Polymernetzwerk hinzugegeben und durch anschließende Trocknung eine NOD-Beschichtung der Fasern erzielt.

In einer Ausführungsform der Erfindung ist die Haut- oder Wundauflage als mehrschichtige Auflage gestaltet, die die folgenden Schichten umfasst:
- eine Trägerschicht,
- eine Schicht enthaltend ein sauer hydrolysierendes Oligomer oder Polymer und ein säureaktivierbares Prodrug, insbesondere eine NOD; und
- optional eine Rückschicht.

In einer besonderen Aspekt stellt die Erfindung somit eine Haut- oder Wundauflage bereit, die zur Verwendung bei der Behandlung von Erkrankungen mittels Auflegen oder Aufkleben auf das zu exponierende Areal geeignet ist.

Die erfindungsgemäße Haut- oder Wundauflage kann hierbei insbesondere zur Anregung des Stoffwechsels von Geweben durch äußerliche Anwendung, im Bereich der Dermatologie zur Behandlung von chirurgischen oder unfallbedingten Wunden, chronischen, nicht- bzw. schlechtheilenden und/oder bakteriell bzw. Pilz-befallenen Wunden sowie zur Behandlung von dermatologischen Erkrankungen aus dem Formenkreis der entzündlichen, immunologisch gesteuerten bzw. Autoimmunerkrankungen verwendet werden. Bevorzugte Beispiele für mögliche Anwendungsgebiete sind wie folgt:
- Behandlung diabetischer Füße und Wunden;
- Behandlung neuropathischer Schmerzen;
- Behandlung von Krampfadern;
- Behandlung von lokalen oberflächlichen oder tiefsitzenden Ischämien und thrombopathischer Erkrankungen von Geweben;
- Behandlung akuter und chronischer Entzündungen der Haut;
- Behandlung von Allergien der Haut;
- Behandlung von parasitären Infektionen der Haut;
- Behandlung der atopischen Dermatitis insbesondere Neurodermitis, Dermatomyositis und Pemphigus vulgaris;
- Behandlung von Wunddefekten, wie dem chronischen diabetisch-neuropathischen Ulcus, Ulcus cruris, Dekubituswunden;
- Behandlung sekundär heilender infizierter Wunden;
- Behandlung primär heilender Wunden, wie ablative Riss- oder Schürfwunden;
- Behandlung größerer Körperareale zur Therapie systemischer Erkrankungen wie z. B. des erhöhten Blutdrucks (Hypertonie) und verwandten hämodynamischen Erkrankungen;
- Behandlung von Patienten mit (Haut)transplantaten;
- Behandlung des diabetischen Schmerzes der unteren Extremitäten (Fuß oder Bein); und
- Behandlung von schlecht perfundierten Lappenplastiken.

Zudem könnte es möglich sein, durch Behandlung größerer Körperareale auch systemische Erkrankungen, wie z. B. den erhöhten Blutdruck (Hypertonie) und verwandte hämodynamische Erkrankungen zu adressieren.

Zum Zweck einer Behandlung wird die Haut- oder Wundauflage auf das zu exponierende Areal aufgelegt und dann bevorzugterweise durch die in der Wunde bzw. Haut vorhandenen Feuchtigkeit die Hydrolyse des sauer-hydrolysierenden Polymers oder Oligomers initiiert. Die Behandlungszeit kann zwischen wenigen Minuten und mehreren Tagen dauern.

In einer Ausführungsform der Erfindung beträgt die Behandlungszeit zwischen 5 min und 3 Tagen, bevorzugt zwischen 5 Stunden bis 2 Tagen und besonders bevorzugt zwischen 20 bis 30 Stunden.

In einer Ausführungsform der Erfindung wird die medizinische Auflage zur Behandlung von Erkrankungen verwendet. Hierbei wird in zweckmäßiger Weise die medizinische Auflage auf den zu exponierenden Areal des Körpers, also bspw. einen Rumpfabschnitt, oder einen Extremitätenabschnitt aufgelegt und dann durch die Säure-induzierte Freisetzung von NO aus der Haut- oder Wundauflage dieses entsprechende Areal dem NO ausgesetzt.

In einer besonders bevorzugten Ausführungsform wird die erfindungsgemäße medizinische Auflage zur Behandlung chronischer Wunden der unteren Extremitäten und hierbei insbesondere bei Diabetikern eingesetzt. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

In einer Ausführungsform der Erfindung wird die medizinische Auflage zur Therapie schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen sowie systemischen Wirkung, die das Risiko der Entstehung von Wunden signifikant mindert bzw. deren Heilung wesentlich beschleunigt. Das mittels der medizinischen Auflage dem Körper in lokal begrenzter Form dem zu behandelnden Extremitätenabschnitt oder dem Rumpfabschnitt zugeführte NO kann daher erfolgreich zur Therapie schlechtheilender Wunden angewendet werden.

In einer besonderen Ausführungsform wird die erfindungsgemäße medizinische Auflage zur Behandlung des diabetischen Schmerzes der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit der medizinischen Auflage von außen dem Fuß und/oder Bein zugeführte NO kann daher erfolgreich zur Therapie diabetischer Fuß- oder Beinschmerzen angewendet werden.

In einer speziellen Ausführungsform der Erfindung wird die erfindungsgemäße medizinische Auflage zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

Die erfindungsgemäße medizinische Auflage kann somit nicht nur zur Behandlung von chronischen oder akuten Erkrankungen eingesetzt werden, sondern auch zur möglichen Prävention solcher Erkrankungen. Sofern nicht anders ausgeführt, umfasst der Begriff "Therapie" oder "Behandlung" alle Maßnahmen zur Linderung, Heilung oder Prävention der hier relevanten Erkrankungen.

Ein derartiges Auflagebehandlung kann in Abstanden von 1, 2, 3, 4, 5, 6, oder 7 Tagen oder sogar mehrmals täglich angewendet werden, wobei ein Wechsel der Auflage nach 1 bis 2 Tagen bevorzugt ist..

In einem weiteren Aspekt stellt die Erfindung eine pharmazeutische Zusammensetzung zur Freisetzung eines pharmazeutischen Wirkstoffs bereit der folgendes umfasst:
o ein Oligomer oder Polymer, das durch Hydrolyse Säure bilden kann, und
o ein säure-aktivierbares Prodrug,
wobei das säure-aktivierbare Prodrug durch die mittels Hydrolyse gebildete Säure aktivierbar ist.

In einer bevorzugten Ausführungsform ist bei der pharmazeutische Zusammensetzung das Prodrug eine pH-labile NO-Vorstufe (NOD).

Die pharmazeutische Zusammensetzung kann hierbei alle bekannten galenischen Formen annehmen und umfasst beispielweise Kapsel, Tablette, Film, Dragee, Gel, Creme, und Aerosol.

Die vorab für die Haut- oder Wundauflage dargestellten Ausführungsformen (insbesondere hinsichtlich der NOD und der sauer-hydrolysierenden Oligomere und/oder Polymere) können ebenso auch für die erfindungsgemäße pharmazeutische Zusammensetzung eingesetzt werden.

In einem weiteren Aspekt stellt die Erfindung ein Verfahren zur Freisetzung eines Wirkstoffs aus einer pharmazeutischen Zusammensetzung bereit und insbesondere zur Freisetzung des Wirkstoffs aus einer Haut- oder Wundauflage, wobei das Verfahren die folgenden Schritte umfasst:
(a) Bereitstellen einer pharmazeutischen Zusammensetzung und insbesondere einer Haut- oder Wundauflage umfassend ein Oligomer und/oder Polymer, das durch Hydrolyse ein saures Milieu erzeugen kann, und ein säure-aktivierbares Prodrug;
(b) Inkontaktbringen des Polymers und/oder Oligomers mit einem Induktor oder Katalysator, so dass dieser die zumindest teilweise Hydrolyse des Oligomers und oder Polymers bewirkt und durch die Hydrolyseprodukte ein saures Milieu entsteht;
(c) Umwandlung des säureaktivierbaren Prodrugs durch das saure Milieu aus Schritt (b) in einen Wirkstoff;
(d) Freisetzen des Wirkstoffs aus der pharmazeutischen Zusammensetzung und insbesondere aus der Haut- oder Wundauflage.

Der im Schritt (b) einwirkende Katalysator oder Induktor kann ein Bestandteil der pharmazeutischen Zusammensetzung sein, er kann aber auch von außen dieser pharmazeutischen Zusammensetzung hinzugefügt werden. So kann er beispielweise durch den zu behandelnden Organismus zur Verfügung gestellt werden.

Weiterhin kann die Haut- oder Wundauflage auch eine Vorrichtung zur Messung der Durchblutung umfassen, der anhand des Therapieerfolges eine besonders gute Steuerung der Behandlungsdauer und/oder Behandlungsintensität erlaubt. Dem Fachmann sind zahlreiche Vorrichtungen zur Messung der Durchblutung bekannt. Beispiele hierfür sind Gefäßtachometer, oder der in der WO 97/46853 offenbarte Mikrosensor.

Als Surrogatparameter für die Hautdurchblutung können weitere vaskulär bedingte Messparameter wie die Rötung der Haut oder die Hauttemperatur dienen, für die entsprechende Messmethoden und -geräte aus dem Stand der Technik bekannt sind.

### BEZUGSZEICHEN

1 Faser aus sauer-hydrolysierendem Polymer
2 Kernfaser aus nicht sauer-hydrolysierendem Polymer
3 NO-Prodrug-Partikel
4 Trägerschicht
5 Gewirk oder Vlies mit Fasern enthaltend die sauer-hydrolysierenden Polymere
6 NO-durchlässige Schutzschicht

### FIGUREN

Die Erfindung wird nachfolgend anhand der Figuren näher erläutert ohne die Erfindung auf dieses zu beschränken. Es zeigen:

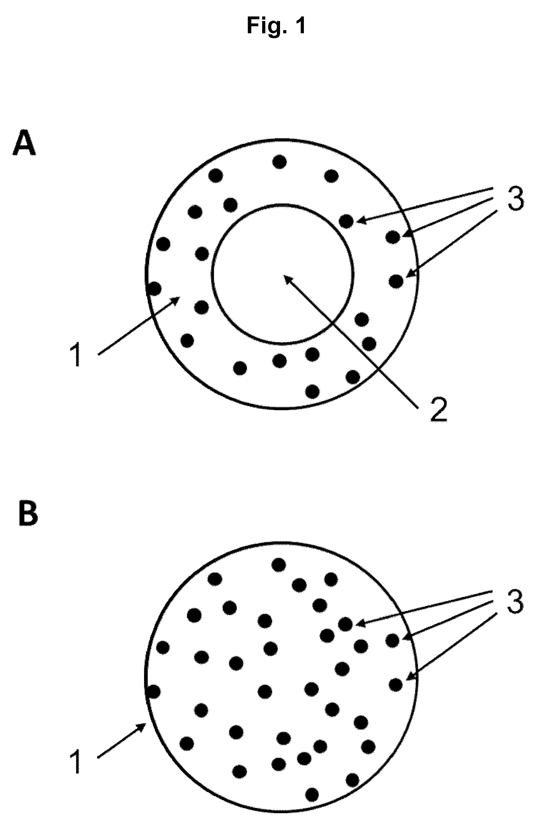

- Fig. 1:: zwei Ausführungsformen einer erfindungsgemäßen Faser: In (A) mit einer Kernfaser aus nicht sauer-hydrolysierendem Polymer (1) und einer Faserhülle aus sauer-hydrolysierendem Polymer (2), welche NO-Prodrug-Partikel (3) enthält. In (B) ist eine Faser aus sauer hydrolysierendem Polymer (2) dargestellt, welche NO-Prodrug-Partikel (3) enthält.

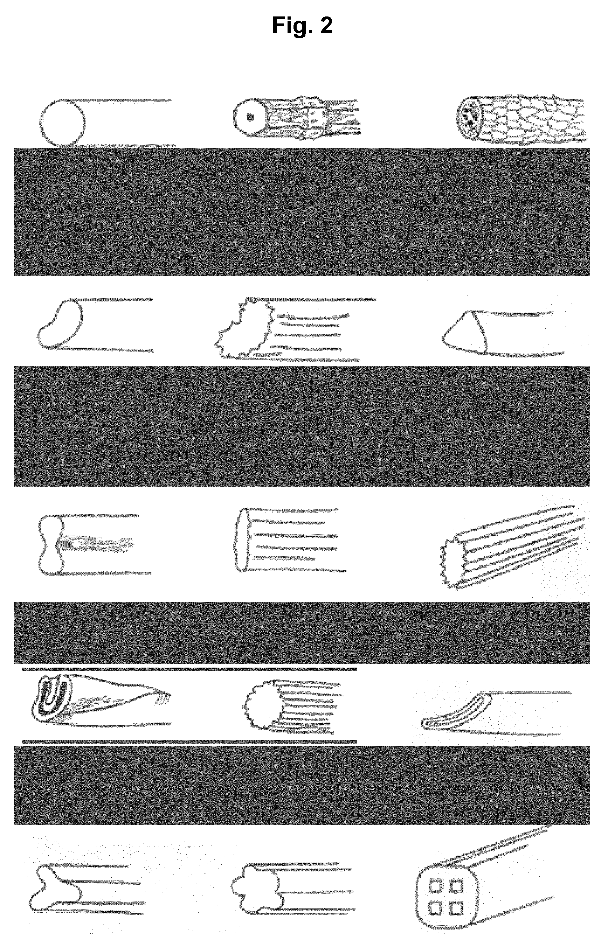

- Fig. 2:: ausgewählte Querschnitte für eine erfindungsgemäße Faser. In einer schematischen Darstellung sind von oben links nach unten rechts dargestellt: Zirkulär mit gleichmäßigem Durchmesser; Polygonal mit Lumen; oval bis rund mit überlappendenden Skalen, oval-flach mit Lumen-Faltung, sternförmig, bohnenförmig, gezahnt bohnenförmig, triangulär mit abgerundeten Ecken, trilobal, lobulär mit Einkerbungen, hantelförmig, abgeflacht, Konzertina-förmig, kollabierter Tubus mit Hohlkern, abgerundetes Quadrat mit vier Hohlräumen.

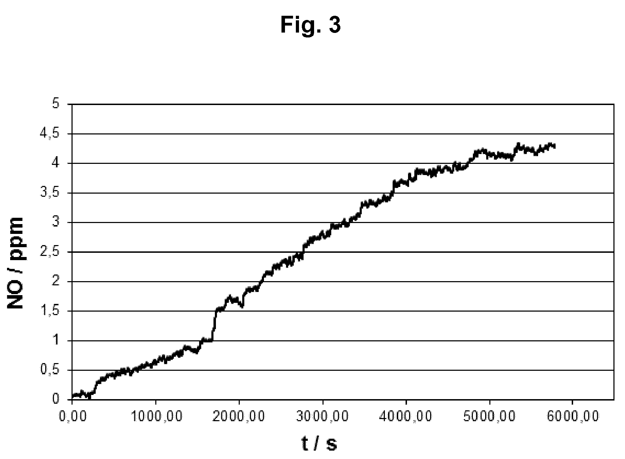

- Fig. 3:: die Bildung von NO über einen Zeitraum von 100 min in einer PLA-Membran nach Zugabe von Wasser, Natriumnitrit und Natrium-L-Ascorbat. Aufgetragen ist die Menge an gebildetem NO in ppm über die Zeit in Sekunden. Zu Details siehe Beispiel 1.

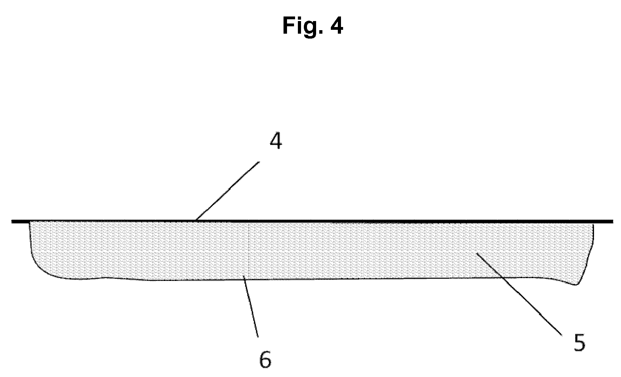

- Fig. 4:: eine erfindungsgemäße Wundauflage mit einem Träger (4) (z.B. als Folie oder Gewebeträger) auf den ein Gewirk oder Vlies (5) aufgebracht ist, das Fasern aus sauer-hydrolysierenden Polymeren oder Oligomeren umfasst und eine abschließende Schutzschicht (6), die durchlässig für NO ist und verhindert, dass das Gewirk oder das Vlies mit der Wunder verklebt.

### BEISPIELE

### I. Generierung von NO mittels sauer hydrolysierendem Polymer in einer Wundauflage

### 1. Materialien:

- NO-Gasanalysegerät Eco physics CLD 822 (ECO PHYSICS AG, Duernten, Schweiz) zur Quantifizierung von NO
- Natriumnitrit (90 mg) und Natrium-L-Ascorbat (90 mg)
- 2ml Aqua dest.
- 15 cm² Polylactid (PLA) Membran (Membran mit Mikroporen von 2-50 µm Durchmesser, Zusammensetzung: Tri-polymer des Polylactids als Hauptbestandteil, darüber hinaus Trimethylencarbonat and ε-Caprolacton (Lactocapromer) als Bestandteile)

### 2. Methode

90mg Natrium-L-Ascorbat und 90 mg NaNO₂ wurde gleichmäßig auf eine 15 cm² große Fläche der PLA-Membran verteilt.

Nach Zugabe von 2ml Aqua dest. wurde die Membran in einer Kammer mit einem Stickstoff- Trägergas überströmt (25ml/min), welches das ausgasende NO der Quantifizierung per CLD zugeführt hat.

Über einen Zeitraum von 100 min wurde kontinuierlich die NO Konzentration mit Hilfe des CLD Systems quantifiziert.

### 3. Ergebnisse

Die Ergebnisse der NO-Quantifizierung mittels CLD sind in der Fig. 3 dargestellt. Es ist ein kontinuierlicher Anstieg der NO-Konzentration zu beobachten, wobei nach ca. 75 Minuten relevante Werte von 4ppm erreicht werden. Durch Anwesenheit des Antioxidans Ascorbat wurde durch Unterdrückung der NO₂-Bildung hochreines NO als Reaktionsprodukt erhalten.

## Patentansprüche

1. Haut- oder Wundauflage umfassend
(a) ein Oligomer und/oder Polymer, das durch seine Hydrolyse ein saures Milieu erzeugen kann, und
(b) ein säure-aktivierbares Prodrug,
wobei das säure-aktivierbare Prodrug durch die mittels Hydrolyse gebildete Säure in der Haut- oder Wundauflage aktivierbar ist.

2. Haut- oder Wundauflage gemäß Anspruch 1, **dadurch gekennzeichnet, dass** sie zusätzlich einen Induktor oder Katalysator der Oligomer- oder Polymer-Hydrolyse umfasst.

3. Haut- oder Wundauflage gemäß Anspruch 2, **dadurch gekennzeichnet, dass** der Katalysator ein Enzym ist, wobei eine Esterase oder Lipase als Enzym bevorzugt ist.

4. Haut- oder Wundauflage gemäß einem der vorherigen Ansprüche, **dadurch gekennzeichnet, dass** das Polymer ausgewählt ist aus der Gruppe enthaltend Polyester, Polyanhydrid und Polyorthoester, wobei ein Polyester bevorzugt ist.

5. Haut- oder Wundauflage gemäß Anspruch 4, **dadurch gekennzeichnet, dass** das Polymer ein Polyester ist, der ausgewählt ist aus der Gruppe enthaltend Poly(D,L-lactid), Poly(D-lactid), Poly(L-lactid), Poly(D,L-glycolid) (PDLLA), Poly(D-glycolid), Poly(L-glycolid), Poly(lactid-Co-Glycolid) (PLGA), Polyhydroxybutyrat, Poly(ε-caprolacton) (PCL), Poly(ε-caprolacton-Co-Glycolid-Co-DL-Lactid) und Poly(p-Dioxanon) (PPDO), Poly(butylensuccinat), Poly(ethylensuccinat) (PES), Poly(butylensuccinat-Co-adipat), Ply(trimethylencarbonat), Poly(propylenfumarat) (PPF) und andere aliphatischen Polyester sowie deren Copolymere einschließlich segmentierter Blockcopolymere aus Polyether und Polyestersegmenten.

6. Haut- oder Wundauflage gemäß einem der Ansprüche 4 oder 5, **dadurch gekennzeichnet, dass** das Polymer als Faser oder als Hüllschicht einer Kernfaser ausgeformt ist.

7. Haut- oder Wundauflage gemäß Anspruch 6, **dadurch gekennzeichnet, dass** die Faser einen flachen, ovalen, runden, dreieckigen, dreilappigen, Y-förmigen, T-förmigen, M-förmigen, S-förmigen, Y-förmigen H-förmigen, sternförmigen oder schneeflockenartigen Querschnitt aufweist.

8. Haut- oder Wundauflage gemäß einem der Ansprüche 6 oder 7, **dadurch gekennzeichnet, dass** sie die Faser als Bestandteil eines Gewebes, Gewirkes oder Vliesstoffes enthält.

9. Haut- oder Wundauflage gemäß einem Ansprüche 1 bis 3, **dadurch gekennzeichnet, dass** das Oligomer ein zyklischer Ester oder ein zyklisches Anhydrid ist, wobei Glycolid oder Lactid bevorzugt sind.

10. Haut- oder Wundauflage gemäß einem der vorherigen Ansprüche zur Freisetzung von Stickstoffmonoxid, **dadurch gekennzeichnet, dass** das säureaktivierbare Prodrug eine pH-labile NO-Vorstufe ist, wobei ein Nitritsalz bevorzugt ist.

11. Haut- oder Wundauflage gemäß einem der vorherigen Ansprüche, **dadurch gekennzeichnet, dass** das säureaktivierbare Prodrug als Mikro- oder Nanopartikel vorliegt, die bevorzugt in der Polymermatrix eingebettet sind.

12. Haut- oder Wundauflage gemäß einem der vorherigen Ansprüche, **dadurch gekennzeichnet, dass** sie ein System umfasst, welches mehrfach oxidierte Stickoxide, Sauerstoffradikalanionen oder Hydroxylradikale abbaut oder neutralisiert, wobei das System bevorzugt ausgewählt ist aus der Gruppe enthaltend Ascorbinsäure, Vitamin E und Derivate hiervon, Thiole und Radikalfänger.

13. Haut- oder Wundauflage gemäß einem der vorherigen Ansprüche, **dadurch gekennzeichnet, dass** sie folgendes umfasst:
(a) eine Trägerschicht
(b) eine Schicht enthaltend
i. ein Oligomer gemäß Anspruch 1 bis 3 oder 9; und/oder
ii. ein Polymer gemäß einem der Ansprüche 1 bis 8; und
iii. ein säureaktivierbares Prodrug gemäß einem der Ansprüche 1, 10 oder 11; und
(c) optional eine Rückschicht.

14. Haut- oder Wundauflage gemäß einem der vorherigen Ansprüche zur Verwendung bei der Behandlung von Erkrankungen mittels Auflegen oder Aufkleben auf das zu exponierende Areal.

15. Haut- oder Wundauflage gemäß Anspruch 14 zur Verwendung bei der Behandlung von Erkrankungen mittels Auflegen oder Aufkleben auf das zu exponierende Areal **dadurch gekennzeichnet, dass** die Behandlung ausgewählt ist aus der Gruppe enthaltend:
(a) Behandlung diabetischer Füße und Wunden;
(b) Behandlung neuropathischer Schmerzen;
(c) Behandlung von Krampfadern;
(d) Behandlung von lokalen oberflächlichen oder tiefsitzenden Ischämien und thrombopathischer Erkrankungen von Geweben;
(e) Behandlung akuter und chronischer Entzündungen der Haut;
(f) Behandlung von Allergien der Haut;
(g) Behandlung von parasitären Infektionen der Haut;
(h) Behandlung der atopischen Dermatitis insbesondere Neurodermitis, Dermatomyositis und Pemphigus vulgaris;
(i) Behandlung von Wunddefekten, wie dem chronischen diabetisch-neuropathischen Ulcus, Ulcus cruris, Dekubituswunden;
(j) Behandlung sekundär heilender infizierter Wunden;
(k) Behandlung primär heilender Wunden, wie ablative Riss- oder Schürfwunden;
(l) Behandlung größerer Körperareale zur Therapie systemischer Erkrankungen wie z. B. des erhöhten Blutdrucks (Hypertonie) und verwandten hämodynamischen Erkrankungen;
(m) Behandlung von Patienten mit (Haut)transplantaten;
(n) Behandlung des diabetischen Schmerzes der unteren Extremitäten (Fuß oder Bein); und
(o) Behandlung von schlecht perfundierten Lappenplastiken.

16. Haut- oder Wundauflage gemäß Anspruch 15, **dadurch gekennzeichnet, dass** sie zur Behandlung chronischer Wunden der unteren Extremitäten von Diabetikern verwendet wird.

17. Pharmazeutische Zusammensetzung zur Freisetzung eines pharmazeutischen Wirkstoffs umfassend:
(a) ein Oligomer oder Polymer, das durch Hydrolyse Säure bilden kann, und
(b) ein säure-aktivierbares Prodrug,
wobei das säure-aktivierbare Prodrug durch die mittels Hydrolyse gebildete Säure aktivierbar ist.

18. Pharmazeutische Zusammensetzung gemäß Anspruch 17 zur Freisetzung von Stickstoffmonoxid, **dadurch gekennzeichnet, dass** es als Prodrug eine pH-labile NO-Vorstufe enthält.

19. Verfahren zur Freisetzung eines Wirkstoffs aus einer pharmazeutischen Zusammensetzung und insbesondere aus einer Haut- oder Wundauflage umfassend die folgenden Schritte:
(a) Bereitstellen einer pharmazeutischen Zusammensetzung und insbesondere einer Haut- oder Wundauflage umfassend ein Oligomer und/oder Polymer, das durch Hydrolyse ein saures Milieu erzeugen kann, und ein säureaktivierbares Prodrug;
(b) Inkontaktbringen des Polymers und/oder Oligomers mit einem Induktor oder Katalysator, so dass dieser die zumindest teilweise Hydrolyse des Oligomers und oder Polymers bewirkt und damit ein saures Milieu entsteht;
(c) Umwandlung des säureaktivierbaren Prodrugs durch das saure Milieu aus Schritt (b) in einen Wirkstoff;
(d) Freisetzen des Wirkstoffs aus der pharmazeutischen Zusammensetzung und insbesondere aus der Haut- oder Wundauflage.
